# EUROPEAN PATENT APPLICATION

(11) **EP 3 150 188 A1**
(43) Date of publication of application: **05.04.2017**
(21) Application number: 16191409.8
(22) Date of filing: 29.09.2016
(51) Int. Cl.: A61K 8/42, A61K 8/92, A61Q 19/00, A61P 17/00, A61K 36/185, A61K 9/00, A61K 31/164, A61K 8/85, A61K 8/39

(54) **A TOPICAL COMPOSITION OF DEXPANTHENOL FOR THE TREATMENT OF DIAPER RASH**

(30) Priority: 30.09.2015 TR 201512005
(71) Applicant: MONTERO GIDA SANAYI VE TICARET A.S., Istinye, Istanbul 34460 (TR)
(72) Inventor: TÜRKYILMAZ, Ali, 34460 Istanbul (TR); YELKEN, Gülay, 34460 Istanbul (TR); GÜNER, Dicle, 34460 Istanbul (TR); AKAY, Resat, 34460 Istanbul (TR)
(74) Representative: Sevinç, Erkan

(57) **Abstract**

The present invention relates to a topical composition of dexpanthenol comprising argan oil.

## Description

### Field of Invention

The present invention relates to a topical composition of dexpanthenol comprising argan oil.

### The background of the invention

Diaper rash is a common form of irritation and inflammation of those parts of an infant's or adult's body normally covered by a diaper. This condition is also referred to as diaper dermatitis, napkin dermatitis, napkin rash and nappy rash. It is caused by incontinence and diaper use. It frequently occurs in areas immediately adjacent to the diapered area. Hydrated skin is more prone to mechanical damage and chafing of the skin since an increased coefficient of friction is observed, and it may allow irritants to penetrate the stratum corneum more easily. Not only occlusion but also a higher pH can be an underlying factor, which induces several enzymes-mediated irritations. Alkalinization of the skin increases skin penetration of microorganism and activates fecal enzymes.

Dexpanthenol (D-panthenol) is an alcoholic precursor of the pantothenic acid, which represents the biologically active substance. The chemical name of Dexpanthenol is 2,4-Dihydroxy-N-(3-hydroxypropyl)-3,3-dimethylbutanamide and its chemical structure is shown in Formula I.

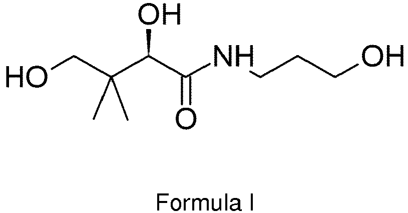

It is used for healing of wounds, stimulates mitotic activity and epithelization, and has inflammatory properties, which is why it is applied topically. Dexpanthenol is absorbed by the skin where it is converted into pantothenic acid (Vitamin B5) and is thus a provitamin of B5. Topical dexpanthenol acts like a moisturizer, improving stratum corneum hydration, reducing transepidermal water loss and maintaining skin softness and elasticity. Its cutaneous regeneration activity results from the activation of fibroblasts proliferation, which is relevant in the healing of wounds and of the injured mucous membrane.

In literature, dexpanthenol was shown to alleviate dry, inflamed skin in an experimental model of skin irritation due to repetitive washing. It improves stratum corneum hydration, stabilizes the epidermal barrier function, and shows an anti-inflammatory effect. Moreover, beneficial effects of dexpanthenol have been observed in patients who have undergone skin transplantation or scar treatment, or therapy for burn injuries and different dermatoses.

Besides the advantages of topical dexpanthenol compositions, it has also disadvantages. Dexpanthenol is almost insoluble in oil. It is accordingly difficult to produce a topical composition comprising a sufficient dissolved concentration of active agent for it to exert its full effect and also to optimize the penetration of the compound into the skin. It is hard to provide a particularly rapid cure because of poor penetration of the active ingredient into the skin.

In addition to ease of release, it is also important that any composition of a pharmaceutically active agent should be stable for long periods of time. Physical, chemical, and microbiological factors play role in the stability of topical compositions. Generally for topical compositions, stability requirements include phase separation, color changes, odor, viscosity (dispensability), preservation, chemical changes (pH, hydrolysis, etc.) and light stability. Other concerns include packaging, weight change, and robustness during manufacture and filling.

In this present invention, argan oil is used in order to increase stability of topical composition of dexpanthenol. While argan oil provides the stability of topical composition, it also increases the skin penetration.

Argan oil is extracted from the fruit of the argan tree (Argania spinosa, Sapotaceae family). It grows in the southwestern part of Morocco. Nowadays, due to its benefits for skin, it is widely used in cosmetic preparations. One of the main reasons that argan oil is beneficial for skin is that it is rich with vitamin A, vitamin E and fatty acids. It is absorbed easily and is non-greasy and non-irritating, which makes it a great natural moisturiser. Research shows that when applied to skin, it heals inflammation while moisturizing and soothing the skin. When applied externally, trocopherol from the vitamin E, helps to enhance cell production while promoting healthy skin.

Patent application FR2553788 discloses a process for the preparation of a lipid extract of the fruit of argan tree. Patent application EP1764085 describes a cosmetic composition comprising argan oil. Patent application US 20150044309 A1 describes an antifungal, antibacterial topical composition comprising argan oil for the prevention and treatment of various skin conditions including diaper rash.

In prior art, there are topical compositions comprising dexpanthenol or argania oil, however, there is no topical composition comprising both dexpanthenol and argania oil. In this present invention, a topical composition of dexpanthenol wherein the composition comprises argania oil is disclosed to overcome stability problems.

### Detailed description of the invention

The present invention provides a topical composition of dexpanthenol comprising argan oil.

In an embodiment, dexpanthenol is used in an amount of between 0.5 - 20.0%, preferably 0.5 - 15.0% and more preferably 1.0 - 10.0% by the weight of total composition.

The main embodiment of this present invention is to obtain a stable topical composition of dexpanthenol. In this invention, argan oil is used in order to increase the stability of topical composition of dexpanthenol. While argan oil enhances the stability of topical composition, it also increases the skin penetration.

For an effective treatment, topical composition of dexpanthenol should meet some stability criterias as indicated above such as optimum pH and viscosity and phase stability. Due to low solubility of dexpanthenol, the topical composition of dexpanthenol shows phase stability problems. The phase stability of a topical composition characterized by the absence of coalescence of the internal phase, absence of creaming and maintenance of elegance with respect to appearance. A topical composition should be homogenous and active agent should be dispersed in composition homogenously. Insoluble substances or complexes cause irritating to the skin or mucosa. Moreover, unstable topical compositions in that phase separation is seen, results lack of uniformity of drug distribution and poses skin penetration problems. In this present invention, to overcome stability problems, argan oil has been used. It has been found that argania oil decreases phase separation of topical composition. By using argan oil, a stable topical composition of dexpanthenol has been developed.

According to one embodiment, argan oil is argania spinosa kernel oil.

According to this embodiment, in order to achieve desired solubility of dexpathenol and stability of the topical composition, argania spinosa kernel oil is used in a specific amount. Argania spinosa kernel oil is present in an amount of between 0.001 - 20.0%, preferably 0.01 - 15.0% and more preferably 0.1 - 5.0% by the weight of total composition.

In this embodiment, the ratio of dexpanthenol to argania spinosa kernel oil which is between 1 - 50 (w/w), preferably 1 - 25 (w/w) and more preferably 1 - 10 (w/w), provides phase stability of topical composition.

In an embodiment, polyglyceryl-3- polyricinoleate (Akoline PGPR) is used to optimize phase separation and provide stability of topical composition comprising dexphantenol.

According to this embodiment, polyglyceryl-3- polyricinoleate is used in an amount of between 1.0 - 20.0%, preferably 1.0 - 15.0% and more preferably 1.0 - 10.0% by the weight of total composition in order to achieve desired effect of Polyglyceryl-3-Polyricinoleate.

In a further embodiment, the ratio of polyglyceryl-3- polyricinoleate to argania spinosa kernel oil 1 - 50 (w/w), preferably 1 - 25 (w/w) and more preferably 1 - 10 (w/w), provides phase stability of topical composition.

In an embodiment of this present invention, said topical composition of dexpanthenol is in the form of diaper rash cream, foam, gel, paste, lotion, emulsion, balm or combinations thereof.

In this embodiment, said topical composition of dexpanthenol is in the form of diaper rash cream.

The topical composition of this present invention is for use in the treatment of diaper rash.

According to a further embodiment, topical composition of dexpanthenol may also comprise natural emmolients, natural humectants, natural antioxidants, natural emulsifiers, natural surfactants, natural preservatives and antimicrobial preservatives.

Suitable emollients are selected from the group consisting of cod liver oil, shea butter, lanolin, jojoba oil, avocado oil, argania spinosa kernel oil, aloe vera oil, prunus amydalus dulcis oil, coco-caprylate/caprate, rosmarinus officinalis oil, triticum vulgare extract, helianthus annuus oil, chamomille oil, rice bran oil, foeniculum vulgare oil, jasminum officinale oil, sesame (sesamum indicum) oil, rosehip, centella asiatica extract, cocoa, jojoba butters, cera alba, fumatrice propolis extract, cremerlin pura and mixtures thereof.

Suitable natural humectants are selected from the group consisting of triticum vulgare oil, oenothera biennis oil, daucus carota oil, jojoba oil, calendula officinalis oil, prunus armeniaca oil, vitis vinifera oil, citrus aurantium oil, foeniculum vulgare oil, jasminum officinale oil, tilia cordata flower extract, chamomille oil, sesame (sesamum indicum) oil, aloe vera oil, rosmarinus officinalis oil, red mandarin oil, shea (butyrosperum parkii) butter, lecithin, panthenol (pro-vitamin b5), glycerin and mixtures thereof.

Suitable natural antioxidants are selected from the group consisting of pythrox LT10-IP (lecithin,ascorbyl palmitate+tocopherol rch extract,sunflower oil), vitamin E (tocopheryl asetate/tocopheroll), ascorbic acid (vitamin C) or ascorbyl palmitat propolis extract, allium cepae (onion) bulb extract, glycyrrhiza glabra (organic licorice) root extract and mixtures thereof.

Suitable natural emulsifiers and natural surfactants (emulgator) are selected from the group consisting of candelilla, carnauba, jojoba, rice bran, beeswax, lanolin, xanthan gum, quince seed, lesitin, glyceryl oleate, glyceryl stearete citrate, castile soap, yucca extract, soapwort, quillaja bark extract, lanolin and mixtures thereof.

Suitable natural preservatives are selected from the group consisting of rosmarinus officinalis leaf extract, melaleuca alternifolia leaf oil, hamamelis virginiana extract, tea tree essential oil, thyme essential oil, grapefruit seed extract, bitter orange extract propolis extract, caprylhydroxamid acid, caprylyl glycol, glycerine and mixtures thereof.

Suitable antimicrobial preservatives are selected from the group consisting of propylene glycol, phenoxyethanol, methyl paraben, propyl paraben and their salts (such as sodium, potassium), sodium benzoate, citric acid, benzoic acid, butylated hydroxytoluene, boric acid, sorbic acid, benzyl alcohol, benzalconium chloride, parahydroxybenzoic acids or butylated hydroxyanisole or mixtures thereof.

In one embodiment, the topical composition comprising;
a. 1.0 - 20.0% Dexpanthenol,
b. 0.001 - 20.0% Argania spinosa kernel oil,
c. 1.0 - 20.0% Polyglyceryl-3- polyricinoleate,
d. 1.0 - 30.0% Emollient,
e. 1.0 - 50.0% Emulsifier and surfactants,
f. 0.001 - 5.0% Antioxidants
g. 0.01 - 5.0% Preservatives by weight of total composition

**Example 1:**

| | **Amount (%)** |
|---|---|
| Zinc oxide | 15.0 |
| Lanolin | 5.0 |
| Beeswax (Cera alba) | 1.0 |
| Cremerlin Pura (vegetable oil) | 3.0 |
| Cod Liver oil | 5.0 |
| Shea Butter (butyrospermum parkii) | 4.0 |
| Pythrox LT10-IP (Lecithin+ascorbyl palmitate+tocopherol rch extract+sunflower oil) | 0.20 |
| Argania spinosa kernel oil | 1.0 |
| Coco-Caprylate/Caprate (CremerCoor Coco 810) | 3.0 |
| Jojoba oil (simmondsia sinensis) | 1.0 |
| Polyglyceryl-3- Polyricinoleate (Akoline PGPR) | 5.0 |
| Triticum vulgare extract | 3.0 |
| Red Mandarin Oil Sfumatrice | 2.0 |
| Dexphantenol | 5.0 |
| Rosmarinus officinalis leaf extract | 0.01 |
| Caprylhydroxamid acid and caprylyl glycol and glycerine (Spektrastat) | 1.0 |
| Hamamelis Virginiana Extract (Witch hazel LG) | 5.0 |
| Water | q.s. |

| | |
|---|---|
| q.s.: quantum sufficient | |

The production of the composition is carried out as follows:
**Oil phase:** Zinc oxide, lanolin, beeswax, cremerlin pura, cod liver oil, shea butter, Pythrox LT10-IP, argania spinosa kernel oil, coco-caprylate/caprate, jojoba oil, polyglyceryl-3- polyricinoleate, triticum vulgare extract and red mandarin oil sfumatrice are mixed at 75-80°C.

**Aqueous phase:** Dexphantenol, rosmarinus officinalis leaf extract, spektrastat and hamamelis virginiana extract are mixed at 75-80°C.

Aqueous phase is added to the oil phase and the mixture is mixed until it cools.

### Example 2:

| **ingredients** | **Amount (%)** |
|---|---|
| Zinc oxide | 40.0 |
| Lanolin | 5.0 |
| Beeswax (Cera alba) | 1.0 |
| Cremerlin Pura (vegetable oil) | 3.0 |
| Cod Liver Oil | 5.0 |
| Shea Butter (butyrospermum parkii) | 4.0 |
| Pythrox LT10-IP (Lecithin+ascorbyl palmitate+tocopherol rch extract+sunflower oil) | 0.20 |
| Argania spinosa kernel oil | 1.0 |
| Coco-Caprylate/Caprate (CremerCoor Coco 810) | 3.0 |
| Jojoba oil (simmondsia sinensis) | 1.0 |
| Polyglyceryl-3- Polyricinoleate (Akoline PGPR) | 5.0 |
| Triticum vulgare extract | 3.0 |
| Melaleuca alternifolia leaf oil (Tea Tree Oil) | 1.5 |
| Dexphantenol | 5.0 |
| Rosmarinus officinalis leaf extract | 0.10 |
| Caprylhydroxamid acid and caprylyl glycol and glycerine (Spektrastat) | 1.00 |
| Hamamelis Virginiana Extract (Witch hazel LG) | 5.0 |
| Water | q.s. |

| | |
|---|---|
| q.s.: quantum sufficient | |

The production of the composition is carried out as follows:
**Oil phase:** Zinc oxide, lanolin, beeswax, cremerlin pura, cod liver oil, shea butter, Pythrox LT10-IP, argania spinosa kernel oil, coco-caprylate/caprate, jojoba oil, polyglyceryl-3- polyricinoleate, triticum vulgare extract and melaleuca alternifolia leaf oil are mixed at 75-80°C.

**Aqueous phase:** Dexphantenol, rosmarinus officinalis leaf extract, spektrastat and hamamelis virginiana extract are mixed at 75-80°C.

Aqueous phase is added to the oil phase and the mixture is mixed until it cools.

## Claims

1. A topical composition of dexpanthenol comprising argan oil.

2. A topical composition of dexpanthenol according to claim 1, wherein dexpanthenol is used in an amount of between 0.5 - 20.0%, preferably 0.5 - 15.0% and more preferably 1.0 - 10.0% by the weight of total composition.

3. A topical composition of dexpanthenol according to claim 1, wherein argan oil is argania spinosa kernel oil.

4. A topical composition of dexpanthenol according to claim 3, wherein argania spinosa kernel oil is present in an amount of between 0.001 - 20.0%, preferably 0.01 - 15.0% and more preferably 0.1 - 5.0% by the weight of total composition.

5. A topical composition of dexpanthenol according to claim 1, wherein the ratio of dexpanthenol to argania spinosa kernel oil which is between 1 - 50 (w/w), preferably 1 - 25 (w/w).

6. A topical composition of dexpanthenol according to claim 1, further comprises polyglyceryl-3- polyricinoleate.

7. A topical composition of dexpanthenol according to claim 1, wherein polyglyceryl-3- polyricinoleate is present in an amount of between 1.0 - 20.0%, preferably 1.0 - 15.0% and more preferably 1.0 - 10.0% by the weight of total composition in order to achieve desired effect of polyglyceryl-3-polyricinoleate.

8. A topical composition of dexpanthenol according to claim 1, wherein the ratio of polyglyceryl-3- polyricinoleate to argania spinosa kernel oil 1 - 50 (w/w).

9. A topical composition of dexpanthenol according to claim 1, wherein the composition is in the form of diaper rash cream.

10. A topical composition of dexpanthenol according to any preceding claims, for use in the treatment of diaper rash.

11. A topical composition of dexpanthenol according to any preceding claims, comprising;
a. 1.0 - 20.0% Dexpanthenol,
b. 0.001 - 20.0% Argania spinosa kernel oil,
c. 1.0 - 20.0% Polyglyceryl-3- polyricinoleate,
d. 1.0 - 30.0% Emollient,
e. 1.0 - 50.0% Emulsifier and surfactants,
f. 0.001 - 5.0% Antioxidants
g. 0.01 - 5.0% Preservatives by weight of total composition.
